# EUROPEAN PATENT APPLICATION

(11) **EP 1 083 497 A1**
(43) Date of publication of application: **14.03.2001**
(21) Application number: 98921811.0
(22) Date of filing: 26.05.1998
(51) Int. Cl.: G06F 17/30

(54) **METHOD OF ESTIMATING PROTEIN FUNCTIONS**

(71) Applicant: Institute of Medicinal Molecular Design, Inc., Tokyo 113-0033 (JP)
(72) Inventor: ITAI, Akiko, Tokyo 113-0033 (JP); TOMIOKA, Nobuo, Mansion Yuki, Room 302, Tokyo 113-0033 (JP); ITAI, Reiko, Tokyo 113-0033 (JP); IMAMURA, Masazumi, 301, Garden-Ct., Midori-ku, Chiba-shi, Chiba 267-0066 (JP)
(74) Representative: Sternagel, Fleischer, Godemeyer & Partner Patentanwälte
(86) International application number: JP9802302
(87) International publication number: WO9962004

(57) **Abstract**

A database comprising information on amino acid sequences of proteins with one or more known biological functions, and further comprising information on importance scores with regard to the appearance of said biological functions added for each amino acid residue constituting said amino acid sequences, and a method of preparing an alignment of a protein stored in the database and a polypeptide with unknown biological function which comprises the steps of calculating a homology score to the coincidence of each constituent amino acid under consideration of the importance score for the appearance of a biological function, and preparing an alignment representing homology of sites where said importance is high.

## Description

### Technical Field

This invention relates to a method for predicting functions of a protein based on amino acid sequence information, where the protein is constituted by the said amino acid sequence. More specifically, the invention relates to a method for effectively predicting, based on amino acid sequence information, biological functions of a protein constituted by said amino acid sequence, such as enzyme activities, by using a specific database available for computer.

### Background Art

Proteins are essential substances for the maintenance of life activities in organisms, and various proteins exist in animals and plants as well as microorganisms, which bear characteristic functions and roles. Proteins can be roughly categorized with their functions into enzymes which catalyze chemical reactions, receptors which are receptor proteins of signal transducing substances, signal transducing proteins which transfer signals themselves, and proteins which bind and transport specific substances, and each can be further subdivided by various functions. For example, enzymes catalyze specific reactions such as enzymes which reduce specific parts of particular substrates and enzymes which hydrolyze proteins.

Proteins are mainly composed of 20 kinds of amino acids, and are polypeptide molecules in which 50 to 1000 amino acids are linked as a chain in various orders by polypeptide bonds. The order of amino acid linkage (called amino acid sequence or primary structure) is different for each protein, and as a result, each protein can exhibit different physiological functions. That is, once a long polypeptide chain is folded into a certain tree-dimensional structure, capture of target molecules (enzyme substrate molecules, receptor substrate molecules and the like) becomes possible and functional groups related to a reaction are placed at appropriate positions, providing a suitable field for appearance of the target biological function. It is easily predicted that a characteristic steric structure is determined from each amino acid sequence and biological functions are determined from the steric structure, however, the inevitability of those relationships is not well explained.

For the study of proteins, methods of determining a whole amino acid sequence from a gene by picking up the gene encoding a protein, after determining the terminal 20 or less residues and synthesizing the corresponding gene sequence, are being utilized instead of the classical methods, in which a protein is isolated and purified using enzyme activity as an index, the molecular weight, constituting amino acid numbers, and numbers of each amino acid are determined, and finally an amino acid sequence is determined. In these studies, proteins with known functions have been on target, however recently, completely reversed orders of studies are conducted in many cases. The reason is the fact that analysis of gene sequences has become quite easy, and consequently, it has become possible to determine an amino acid sequence of a protein from the gene without isolating the protein.

Consequently, proteins are rapidly increasing for which only amino acid sequences are predicted and whose biological functions remain unknown. Since the biological function of a protein appears based on the steric structure, trials have been made to predict biological functions of such proteins with unknown functions by analyzing the steric structures using crystallographic analysis and NMR analysis. However, for these structural analyses, much larger amount and also highly purified samples are required compared with biochemistry works. Since biological functions are not always predicted from the steric structures and even predicted biological functions are not necessarily important ones, there is also a problem that investment efficiencies of such studies are awfully poor. Therefore, it is desired earnestly to develop a method of predicting biological functions of a protein possessing a certain amino acid sequence before determining its steric structure. If such a method is developed, it will be expected to contribute a great deal to the protein study and genomic study.

Steric structures and biological functions are closely related, and thus steric structure information on proteins with known functions is useful for various purposes, not only for elucidating mechanism of the functions. Three-dimensional coordinates of proteins or complexes with ligand molecules are stored in the Protein Data Bank (Brookhaven National Laboratories, U.S.A.), which is accessible throughout the world. At present, the number of the structures stored is about 5,000, but considering independent proteins excluding the difference in species and mutants, the number becomes about 400 to 500. Although the number of proteins that are analyzed crystallographically is increasing with accelerating speed due to the propagation of analysis techniques and the progress of isolation and purification techniques of proteins, proteins whose structures are not yet solved are overwhelming majority at present.

It is possible to utilize modeling as a means to predict steric structures of proteins and modes of interactions with ligand molecules, besides crystallographic analysis and NMR analysis. When steric structures of similar proteins with moderately high homology of amino acid sequences have already been analyzed, a steric structure based on the correspondence of amino acid residues can be constructed by performing modeling using the structures as templates. This method has advantages that there is no need to obtain samples, and that the method is generally performed interactively on the computer graphics screen. For instance, for those amino acids that are not identical, it is conducted by replacing the side chains. Although there are problems such as the conformation of side chains and the insertion or deletion of amino acids of main chains, reliability of the predicted structures is governed by the degree of homology of the amino acid sequences and it is possible to treat it like crystal structures almost similarly.

A method of making a correspondence between amino acid sequences of two or more kinds of proteins (alignment) such that the kinds of amino acids match as many as possible, is frequently used for the purpose of examining similarity and difference between species and families as well as for modeling. In the conceptual technique of alignment, a corresponding position with the best coincidence score is found while the sequence is subjected to sliding one by one relative to the other. Actually, however, precise consideration and repeated operations are necessary, because possible correspondences between the sequences are unlimited, and thus extremely complicated operations are necessary in order to obtain an accurate result. For example, the degree of coincidence of amino acids with whole sequences can not simply be taken as a score, because there are often insertions or deletions in one sequence, and thus it is necessary to find a partial sequence which coincides well locally, and also it is necessary to calculate coincidence scores in a unit of certain number of residues (window). In some cases, it is necessary to calculate scores by regarding not strictly identical but similar amino acids as homologous.

Generally, when the homology is low, there is a problem that an alignment is not determined unanimously and still has some uncertainty. However, at present, alignment is the simplest and easiest method of estimating the functions of a protein with the amino acid sequence only as a clue, since proteins with high similarity of the amino acid sequences can be found out from groups of proteins whose functions are known. For these reasons, trials of partially automating the complicated operations of alignment by using a computer have been made. For example, as matching methods of amino acid sequences, FASTA (Pearson W. R. and Lipman, D. J., Proc. Natl. Acad. Sci. USA, 85, pp.2444-244, 1988) and BLAST (Altschul, S. F. et al., J. Mol. Biol., 215, pp.403-410, 1990) are known. These methods are suitable for rapid examination of the existence of a short specific sequence in a long target sequence. However, when long sequences are compared with each other or when sequences with low homology and fragmental coincidence are treated as search targets, judgment of similarity and extraction of similar parts are extremely difficult and accuracy of judging the homology is low. Therefore, these methods are not adequate for the purpose of alignment and prediction of protein functions, and thus a development of a rapid method with better accuracy has been desired.

### Disclosure of Invention

An object of the present invention is to provide a method for predicting functions of a protein based on amino acid sequence information, where the protein is constituted by the said amino acid sequence. More specifically, the object is to provide, when information on amino acid sequences is only available, a method for effectively searching homology to amino acid sequences of proteins with known biological functions with a computer using a specific database, and then searching biological functions of the protein constituted by said amino acid sequence accurately and rapidly.

As a result of the inventors' earnest effort to solve the above-mentioned object, it was discovered that protein functions can be predicted extremely rapidly and accurately from an amino acid sequence when the database with the following characteristics is employed.

The present invention thus provides a database comprising information on amino acid sequences of proteins with one or more known biological functions, and further comprising information on importance scores concerning the appearance of said biological functions which is added to each amino acid residue constituting said amino acid sequences.

This database, for instance, can be used to predict functions of a protein with unknown biological functions based on the homology of amino acid sequences. According to a preferable embodiment, as information on the amino acid sequences of proteins with known biological functions, importance scores concerning the binding between the proteins and ligand molecules or concerning the appearance of biological functions are added to each amino acid residue constituting the said amino acid sequences, by using the amino acid sequences of proteins for which information on the steric structure such as the three dimensional structures of protein is available. These databases, in general, can be stored in various media such as floppy disks, CD-ROM, magnetic tapes, and optical disks.

From other point of view, the present invention provides a method of preparing an alignment of a protein in the above-mentioned database (referred to as "template protein" in the specification) and a polypeptide with unknown biological functions (referred to as "target protein" in the specification), which comprises the steps of calculating homology measure for the coincidence of constituting amino acids under consideration of the importance scores concerning the appearance of biological functions, and preparing an alignment which represents the homology of regions where the said importance is high.

A preferred embodiment of the aforementioned method comprises a step of searching correspondences with high homology regarding the protein in the above-mentioned database and the target protein, by using group sequences containing two or more continuous amino acid residues that are highly important for the appearance of the biological functions. Furthermore, other preferred embodiment includes a method comprises a step of obtaining a final score of homology from the above-mentioned alignment for one of the proteins in the database and the target protein, and a method comprises a step of estimating one or more proteins most similar to the target protein with regard to the biological functions based on the final scores for all the proteins stored in the database. These methods have characteristics that, even if homology of the whole protein is low, proteins having high homology in regions related to the biological functions can be extracted and functions of the target protein can be rapidly and very accurately estimated.

### Brief Explanation of Drawings

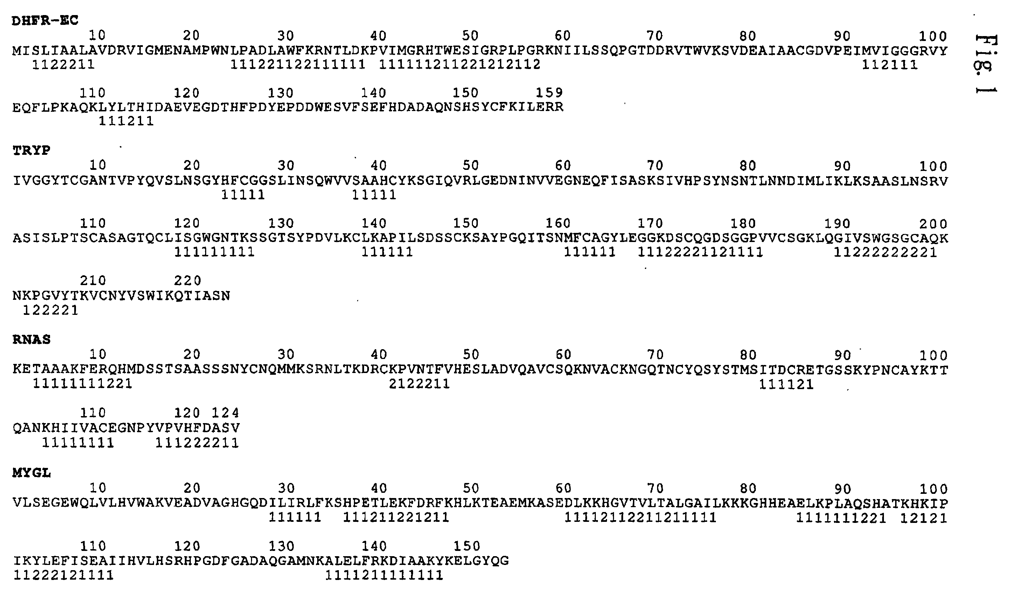

Figure 1 shows information on amino acid sequences with the importance scores concerning the appearance of biological functions added for each amino acid residue constituting the amino acid sequence, for 4 kinds of proteins with known biological functions and steric structures. Symbols in the figure indicate dihydrofolate reductase from E. coli (DHFR-EC), trypsin from bovine (TRYP), ribonuclease from bovine (RNAS), and myoglobin from whale (MYGL), and amino acid residues are indicated by one-letter symbols.

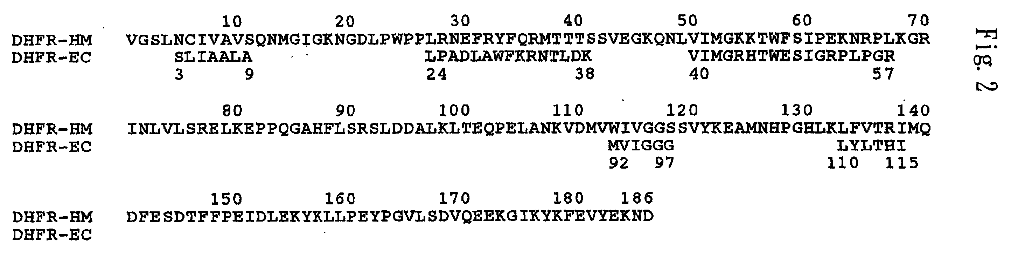

Figure 2 shows an alignment of the target protein (DHFR-HM) and DHFR-EC that was extracted as a template protein yielding the highest SSS score to the target protein. The symbols in the figure indicate dihydrofolate reductase from human (DHFR-HM), and dihydrofolate reductase from E. coli (DHFR-EC) respectively, and first row indicates the amino acid numbers of DHFR-HM, second row indicates the amino acid sequence of DHFR-HM, third row indicates the partial sequences from the amino acid sequence of DHFR-EC, and fourth row indicates the amino acid numbers of partial amino acid sequence of DHFR-EC.

### Best Mode for Carrying out the Invention

Database of the present invention is characterized that it includes information on amino acid sequences of proteins with one or more known biological functions, and that it includes information on importance scores concerning the appearance of biological functions added to each amino acid residue constituting the said amino acid sequences. Proteins to be stored can be, for example, any protein insofar as that one or more biological functions like enzyme actions and receptor actions are known and that all amino acid sequences are known. Proteins whose steric structures of proteins or ligand-binding sites have already been elucidated or predicted, or easily predictable, are preferable. In the database, it is desirable to contain information on the protein with known steric structures as much as possible. For instance, three-dimensional coordinates of proteins or complexes with ligand molecules are stored in the Protein Data Bank (Brookhaven National Laboratories, U.S.A.), where information on about 5,000 proteins (about 400 to 500 considering independent proteins excluding the difference in species and mutants) is available, and can be used suitably to make a database of the present invention.

In the database of present invention, it is characterized that, based on the amino acid sequences constituting proteins with known biological functions, importance scores concerning the appearance of the said biological functions are added to each amino acid. As information to be stored for each protein, examples include, for example, name of the protein, species, organ and apparatus, subtype, kind of function, sub-classification of the function (for example, for enzymes, enzyme action such as protein degradation and reduction), enzyme classification number (EC number), ligand molecules related to the enzyme reactions or biological functions (enzyme substrate, receptor substrate, coenzyme, metal ion, and the like), source of the steric structure (for example, X-ray crystallography, NMR analysis, modeling based on information on similar proteins with similar biological functions), main bibliographic references, reference number of other databases, target region ligands, and whole amino acid sequences. However, information is not limited to these examples, and some information may be added or deleted appropriately.

In addition to the above-mentioned information, the database of the present invention includes information on importance scores concerning the appearance of the said biological functions, which is added to each amino acid residue constituting the said amino acid sequences. Importance scores are specified by giving numbers or other symbols, for example, 0 for no importance and 10 for extremely high importance. Preferably, it is general to calculate the scores considering two or more elements related to the importance. To the database of the present invention, it is further possible to add information such as existence of continuous amino acid residue sequence (1 to n) that contributes to the appearance of the biological functions (i.e. score is not zero), method of the scoring, sum of the scores, scale factors for normalizing the sum of the scores among proteins. However, information to be added is not limited to these examples, and appropriate information may be added or deleted. When multiple biological functions or multiple ligand molecules are known for one protein, it is preferable to store information for each, respectively.

In the following descriptions, procedures will be demonstrated for giving importance scores concerning the appearance of biological functions for each amino acid residue constituting amino acid sequences of proteins. The descriptions are presented only as examples and should not be interpreted in any limiting sense. Furthermore, the database of the present invention is not limited to those produced by these procedures. In the following descriptions, examples of indicating the importance scores by numerical values will be explained, in which zero is given to those without any importance and greater numbers are given to those with increasing importance. However, it should be recognized that the scoring method is not limited to these examples.

### (a) In case crystallographic analysis of protein complexes including ligand molecules of small molecular weights has been conducted:

For proteins stored in the Protein Data Bank whose three-dimensional structures in complex with small-molecular-weight ligand molecules such as an enzyme substrate, a receptor substrate or an inhibitor have been analyzed, a distance of each amino acid residue from the ligand molecule can be calculated and importance scores depending on the distance of each amino acid residue are given. As the small-molecular-weight ligand molecules, any molecules may be employed such as, for example, an organic compound that is pharmacologically active, an enzyme substrate, and a metal ion. For instance, a score of 1 can be given to any amino acid residue which is within 10 Å from any atom of the ligand molecule (for example, Ca atom which becomes a ligand or one atom in the side chain of the ligand molecule), a score of 2 if it is within 8 Å, 4 if it is within 6 Å, and 0 if it is more than 10 Å.

### (b) In case crystallographic analysis of unbound protein has been conducted:

For proteins for which crystallographic analyses have been performed without ligand molecules, if structural region related to biological functions (such as enzyme activities) can be predicted from various experiments, it is possible to give numerical values to the neighboring amino acid residues depending on the distance, as is performed in above (a). Even if the correspondence between the biological function and steric structure is not established, insofar as the biological functions are clear, amino acid residues related to the biological functions can be extracted by searching distinguished cavities while the drawing of steric structure of the protein is being rotated on computer graphics screen.

### (c) Structurally conserved region:

For proteins having the same biological functions, if analytical results can be utilized as for the steric structures of two or more kinds such as subtypes with different amino acid sequences and proteins from different species, a structurally conserved region can be extracted by superposing those structures, and high numerical values can be given to the amino acid residues contained in those regions.

### (d) Modeling:

In case the steric structures of proteins have not been analyzed, it is possible to give importance scores based on the modeling structure constructed on the basis of the steric structures of homologous proteins that are known to have practically the same biological functions. It is known that reliability of the modeling structure is high, for example, for those cases such as receptor subtypes, isozymes, proteins of the same family, proteins of different species with the same functions whose amino acid sequences have high homology. The method of giving importance scores, for example, may be conducted similarly to the above-mentioned techniques.

### (e) Biochemical experiment and genomic experiment:

High importance scores can be given to the amino acid residues that are predicted to be important for the appearance of the biological functions from biochemical experiments and the like, and to the amino acid residues that are predicted to be essential for the appearance of the biological functions such as enzyme reactions from the genomic amino acid conversion experiment (point mutation and the like). In enzyme reactions, for example, large numerical values can be given to amino acid residues that play catalytic role, in addition to the evaluation from the bonding with ligand molecules.

### (f) Protein as macromolecular ligand molecule

Generally, proteins in which binding with a small ligand molecule is essential for its function have cavities that bind stably with the ligand molecules. On the other hand, for proteins to which macromolecular ligand molecules like proteins bind, it is frequently observed that they bind with receptor proteins through the molecular surface without forming distinguished cavities, and there are cases where the receptor proteins do not have distinguished cavities. For example, in the case of cytokines which become macromolecular ligand molecules themselves, large numerical values may be given to the amino acid residues in the epitope region which are predicted by using monoclonal antibodies.

By employing a combination of one or more kinds of techniques exemplified above, and further by adding appropriate techniques, if required, importance scores concerning the appearance of the said biological functions can be given to each amino acid residue constituting the amino acid sequences of proteins with known functions, and information on the amino acid sequences attached to the importance scores can be prepared. As for the relationship between amino acid residues and appearance of biological functions, it should be understood that various criteria can be utilized for scoring such as those whose relationship can be predicted to some extent, as well as those whose relationship has been fully proved, for example, by the above-mentioned method (a).

For instance, information can be collected on the amino acid sequence attached to the importance scores for as many proteins as possible such as those with known crystal structures and those predicted to have similar steric structures from biological functional points of view, and then a database of the present invention can be constructed by storing the information in a certain format usable by computers. For this purpose, depending on the quantity and quality of the information on each protein, scores may be given to each protein based on appropriate different criteria. However, there are some cases where the scoring method and a scaling factor used for normalization of the total scores among the proteins need to be added to the database. While it is possible to input the above-mentioned information manually according to a certain method, it is generally effective to perform it by using a certain program on computer graphics screen.

According to the method of the present invention, using the above-mentioned database, an alignment can be prepared concerning the template protein whose information is stored in the database and a target protein with unknown biological functions in such a manner that the homology score calculated from the importance scores of amino acid residues becomes maximum, then similar alignments can be prepared for more than 2 template proteins, or preferably all template proteins, in the database. Subsequently, a template protein with the highest score is selected by comparing homology scores among the template proteins. It can be estimated that the template protein thus selected has high similarity of steric structures to the target protein and has practically the same biological functions.

Above method, in general, is performed by taking out information on the template proteins in the above-mentioned database of the present invention one by one, and by performing an alignment to the amino acid sequence of the target protein. If the information on the amino acid sequence of the target protein is directly available, that information may be input and utilized, and if only the information on the genomic sequence encoding the target proteins is available, it is necessary to use information on the amino acid sequence interpreted from the information of its nucleic acid sequence.

As an example of a preferable method of the present invention includes a method of searching a correspondence with high homology concerning the template proteins and the target protein, by using group sequences comprising 2 or more continuous amino acid residues in the amino acid sequence of template proteins (continuous amino acid residues with scores other than zero) that contribute to the biological functions. However, the alignment procedure is not limited to this method, and may be performed by any method available for those skilled in the art.

By the above-mentioned method which employs group sequences, a homology score for each group is obtained by sliding the group sequence one by one against the amino acid sequence of the target protein, and thereafter, a correspondence of each group sequence to the amino acid sequence of the target protein is determined in such a manner that a total score of all group sequences becomes best while considering factors such as the linkage order and lengths of the group sequences as necessary. This procedure can be performed for all template proteins in the database, and one or more proteins having high total scores can be extracted. It is highly possible that the target protein possesses practically the same biological functions as the template proteins thus extracted.

As for the score of homology, for example, if a group sequence in the template protein coincides with the corresponding amino acid residue in the target protein sequence, it is easy to transfer the importance score to the coincided amino acid residue and simply sum them up. However, in order to prepare an alignment emphasizing the coincidence of amino acids with high importance scores, each importance score may be processed further by one or more functions and then used. Upon preparation of alignments between the target protein and all template proteins contained in the database, total homology scores among the different alignments may be compared. Generally, however, it is desirable to calculate scale factors for normalization of importance scores for each template protein and store them in the database so that goodness of homology can be compared by the sum of the importance scores even for template proteins of different sizes. At the step where alignment between the target protein and each template protein is completed and homology scores are calculated, final scores can be obtained by multiplying the homology score of each template protein by the corresponding scale factor, and goodness or badness of homology among the template proteins can be determined.

In the same kind of proteins existing in different species, for example, aspartic acid and glutamic acid, that have carboxyl group in common and whose side-chain lengths differ only by one carbon atom, sometimes play the same role on similar positions in amino acid sequences. In such cases, it is appropriate to regard these amino acid residues as being coincided, upon judgments of the coincidence of amino acid residues. Furthermore, although amino acid residues such as leucine, isoleucine, and valine differ in the shape and size (bulkiness), they have similar characteristics from a viewpoint of hydrophobicity. Therefore, upon evaluation of the homology of amino acid sequences, it is desirable to use correspondence table that grades the similarity of amino acid residues in order to reflect the existence of these analogous amino acid residues. Although any measures may be used for the similarity of amino acid residues, PAM250 (Dayhoff, M.O., et al., Atlas of Protein Sequence and Structure, Dayhoff, M. O. Ed., Vol. 5, Suppl. 3, pp.345-352, NBRF, Washington, 1978) and BLOSUM (Henikoff, S. and Henikoff, J. G., Proc. Natl. Acad. Sci. USA, 89, pp.10915-10919, 1992) are available examples as correspondence tables describing the similarity.

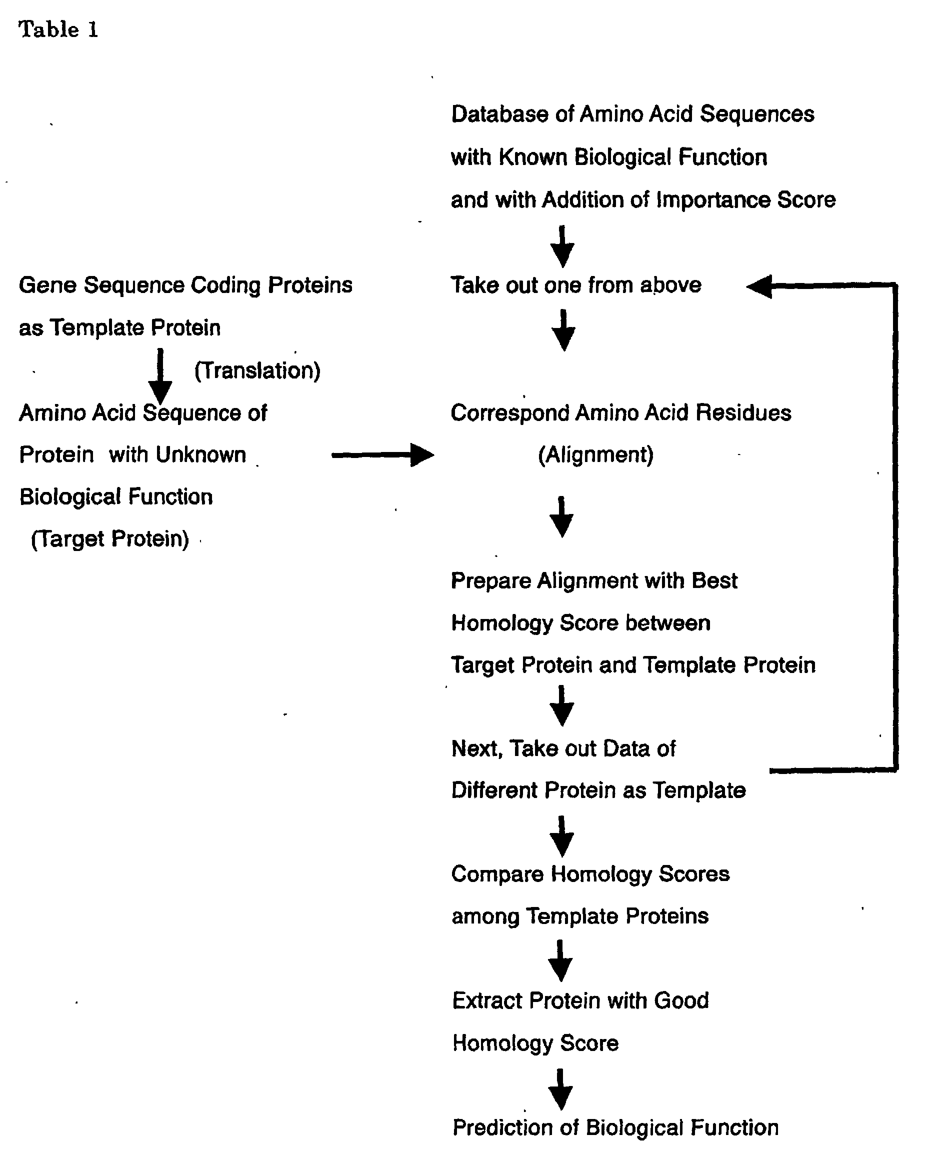

An example of the method of the present invention is shown above as a schematic chart. For example, the method of the present invention may be a method including the following processes. However, a method of the present invention is not limited to these methods, and it should be understood that one or two or more appropriate processes may be added, if necessary, in addition to the processes employed in these methods, and that there are occasions in which one or two or more processes may be omitted, if desired. It should be also noted that such modified or altered methods are all included within the scope of the present invention.
(1) Process of obtaining amino acid sequence of a target protein;
(2) Process of selecting one template sequence from the above-mentioned database;
(3) Process of taking out partial sequences *a, b, c, d,e,* ... ,*n,* ..., with importance scores greater than a certain value, from amino acid sequence of the template protein, for example, from N-terminal successively (length of each partial sequence is represented as *la, lb, lc, ld, le,* ..., *ln,* ...);
(4) Process of placing the partial sequence *a* at the first position of the target sequence and calculating homology score *S(a)i* while sliding the amino acid residues one by one (add importance scores of amino acid residues coincided as a homology score);
(5) Process of placing the partial sequence *b* at the (*l+la*)-th position of the target sequence and calculating homology score *S(b)i* while sliding the amino acid residues one by one (add importance score of amino acid residues coincided as a homology score);
(6) Processes of calculating homology scores *S(n)i* similarly for *c, d, e,* ..., *n,* ...;
(7) Process of determining corresponding positions of partial sequences so that homology SS of all partial sequences become the highest, considering the order *a, b, c, d, e,* ..., *n,* ... and the numbers of amino acid residue of each;
(8) Process of multiplying SS by scale factors to give SSS;
(9) Process of obtaining SSS by the above procedures for all template proteins in the database; and
(10) Process of extracting proteins with high SSS values.

### Example

The present invention is explained more specifically by referring to the following examples. However, the scope of the present invention is not limited to the following examples.

### Example 1: Preparation of database

A database was prepared which comprised four kinds of proteins with known biological functions and known steric structures which are dihydrofolate reductase from E. coli (DHFR-EC), trypsin from bovine (TRYP), ribonuclease from bovine (RNAS), and myoglobin from whale (MYGL). Each crystal structure was obtained from the Protein Data Bank (Brookhaven National Laboratories, USA). Information was prepared on the amino acid sequences with importance scores concerning the appearance of each biological function added to each amino acid residue constituting each amino acid sequence, giving a score of 2 when any constituting atom of an amino acid residue is located within 4 Å from any atom of the ligand molecule (inhibitor or coenzyme), score of 1 for the range between 4 to 10 Å, score of 0 for others. Figure 1 shows the amino acid sequence of each protein and the results of the scoring.

### Example 2: Prediction of biological functions

Biological functions of a target protein was predicted by the method of the present invention, by taking dihydrofolate reductase from human (DHFR-HM) as the target protein and using the above-mentioned database. Although DHFR-HM is a protein with known biological functions and known steric structure, the analysis was performed under assumption that the biological functions and steric structure were unknown. Partial sequences with a score of 1 or more for each amino acid sequence of the template proteins in the database was taken out, and homology score S was calculated by sliding residue one by one against the amino acid sequence of the target protein, and an alignment position with the highest value of S score was determined.

For the calculation of S score, the correspondence table BLOSUM62 was used which concerns similarity of amino acids, and a method was employed in which homology scores was obtained from the table, which corresponded to sets of amino acid residues between the partial sequence and the amino acid sequence of the target protein, and a summation of the product of the score for each residue of the partial sequence and the similarity scores was taken over the length of the partial sequence. Homology SS of all partial sequences was calculated by obtaining the largest value of S scores for each partial sequence and by taking a summation of those values. In order to compensate for the difference in lengths of the sequences used in the homology determination, reciprocal numbers of sums of scores for all partial sequences as scale factors was used, and final scores SSS was calculated by multiplying SS by the scale factors. Consequently, as shown in Table 1, when DHFR-EC, TRYP, RNAS, MYGL were employed as template proteins, DHFR-EC gave the highest score SSS, and it was predicted that the target protein (DHFR-HM) is similar to DHFR-EC and has activities of dihydrofolate reductase. Figure 2 shows the alignments of DHFR-HM and DHFR-EC.

**Table 2**

| Protein | SSS Score |
|---|---|
| DHFR-EC | 1.82 |
| TRYP | 1.09 |
| RNAS | 1.22 |
| MYGL | 0.61 |

### Industrial Application

The database of the present invention is useful for prediction, based on the information on amino acid sequences, of biological functions of proteins constituted of those amino acid sequences. The method of the present invention is useful for accurate and rapid search of biological functions of proteins comprising the amino acid sequences using said database.

## Claims

1. A database comprising information on amino acid sequences of proteins with one or more known biological functions, and further comprising information on importance scores regarding appearance of said biological functions added for each amino acid residue constituting said amino acid sequences.

2. The database according to claim 1 which is utilized to predict function of a protein with unknown biological function based on homology of amino acid sequences.

3. The database according to claim 1 or claim 2 which is prepared by using amino acid sequences of proteins for which information on protein steric structures is available as the information on amino acid sequences of proteins with known biological functions.

4. The database according to any one of claims 1 through 3 which is stored in a storage medium.

5. A method of preparing an alignment of a protein stored in the database according to any one of claims 1 through 3 and a polypeptide with unknown biological function which comprises the steps of:
calculating a homology score to the coincidence of each constituent amino acid under consideration of the importance score for the appearance of a biological function, and
preparing an alignment representing homology of sites where said importance is high.

6. The method according to claim 5, which comprises the step of searching correspondence with high homology concerning the protein in the database and the target protein by using group sequences comprising two or more continuous amino acid residues of high importance with regard to the appearance of the biological function.

7. The method according to claim 5 or claim 6, which comprises the step of obtaining a final score of homology from the alignment regarding one of the proteins in the database and the target protein.

8. The method according to claim 7, which comprises the step of predicting one or more proteins most homologous to the target protein with regard to the biological function based on the final scores for all the proteins in the database.
